(19) Europäisches Patentamt
European Patent Office
Office européen des brevets

(11) Publication number: **0 061 798**
**B1**

(12) **EUROPEAN PATENT SPECIFICATION**

(45) Date of publication of patent specification: **20.08.86**

(21) Application number: **82200303.4**

(22) Date of filing: **09.03.82**

(51) Int. Cl.$^4$: **C 07 D 249/08,**
C 07 D 401/06,
C 07 D 413/06, A 01 N 43/653

# (54) Novel antimicrobial triazole derivatives.

(30) Priority: **27.03.81 US 248634**

(43) Date of publication of application:
**06.10.82 Bulletin 82/40**

(45) Publication of the grant of the patent:
**20.08.86 Bulletin 86/34**

(84) Designated Contracting States:
**AT BE CH DE FR GB IT LI NL SE**

(56) References cited:
**GB-A-1 244 530**

(73) Proprietor: **JANSSEN PHARMACEUTICA N.V.**
**Turnhoutsebaan 30**
**B-2340 Beerse (BE)**

(72) Inventor: **Sturm, Elmar**
**Klusstrasse 66**
**CH-4147 Aesch (CH)**
Inventor: **Meyer, Alfred**
**Bristenweg 6**
**CH-4054 Basel (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 061 798 B1

**Description**

The subject invention is concerned with 2'-amino-2'-phenylethyl-1H-1,2,4-triazoles of the formula (I), as well as with their phytopharmaceutically acceptable acid addition salts with inorganic and organic acids, and their metal complexes. The invention is further concerned with the preparation of such compounds, with antimicrobial compositions which contain a compound of formula (I) as an active ingredient, and with the use of compounds of formula (I) for combating plant diseases. The invention is further concerned with enamines and azomethines prepared as intermediates and with a novel method of preparing the corresponding ketones.

In the formula (I)

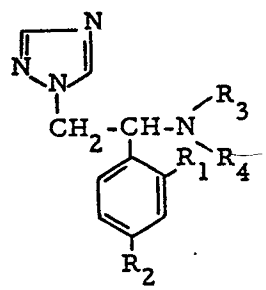

(I)

$R_1$ is a member selected from the group consisting of hydrogen, halo, $C_1$—$C_4$-alkyl, $C_1$—$C_4$-alkyloxy, and $C_1$—$C_2$-haloalkyl;

$R_2$ is a member selected from the group consisting of hydrogen, halo, $C_1$—$C_4$-alkyl, $C_1$—$C_4$-alkyloxy, $C_1$—$C_2$-haloalkyl and phenyl;

$R_3$ is a member selected from the group consisting of hydrogen, $C_1$—$C_{12}$-alkyl, $C_3$—$C_8$-cycloalkyl which is optionally substituted with $C_1$—$C_4$-alkyl, and aralkyl with maximum 2 carbon atoms in the alkyl part and optionally substituted on the phenyl nucleus with halo or nitro;

$R_4$ is a member selected from the group consisting of hydrogen and $C_1$—$C_4$-alkyl; whereby

$R_3$ and $R_4$ may, together with the nitrogen atom to which they are attached, form a 3- to 7-membered saturated heterocyclic ring which may optionally be interrupted by oxygen, sulfur or $NR_5$ and optionally substituted with $R_6$, whereby $R_5$ and $R_6$ are each independently $C_1$—$C_4$-alkyl.

The term "halo" comprises fluoro, chloro, bromo and iodo. Alkyl means, depending on the number of carbon atoms methyl, ethyl, propyl, isopropyl, butyl, isobutyl, sec. butyl, tert. butyl and the different isomers of amyl, hexyl, heptyl, octyl, nonyl, decyl, undecyl and dodecyl.

$C_1$—$C_4$-alkyloxy are methoxy, ethoxy, propyloxy, i.-propyloxy, and the four isomers of butyloxy.

Under $C_1$—$C_2$-haloalkyl are understood, for example, chloromethyl, dichloromethyl, trichloromethyl, 2,2,2-trichloroethyl, perchloroethyl, perfluoroethyl, 1,1,2,2-tetrafluoroethyl, fluoromethyl, difluoromethyl and in particular trifluoromethyl.

By the term $C_3$—$C_8$-cycloalkyl are covered e.g. cyclopropyl, cyclobutyl, cyclopentyl, cyclohexyl, cycloheptyl, cyclooctyl, but also bicyclic ring systems such as 2,2,1-bicycloheptane and 2,2,2-bicyclooctane.

Aralkyl represents, inter alia, benzyl, 2-phenylethyl and 1-phenylethyl. Examples of inorganic acids are hydrochloric, hydrobromic, hydroiodic, sulfuric, phosphoric, phosphorous and nitric acid. Examples of organic acids are, acetic, trichloroacetic, oxalic, malic, maleic, fumaric, tartaric, benzenesulfonic and methanesulfonic acid.

Metal complexes of the formula I comprise the basic organic molecule, and an inorganic or organic metal salt, e.g., hydrohalides, nitrates, sulfates, phosphates, tartrates etc. of copper, manganese, iron, zinc or other metals. The metal kations may thereby exist in their different valencies.

The above enumerations have no limiting meaning.

Imidazole derivatives of acetophenone with plant-fungicide activity are described in D.O.S. 2,604,487.

Aminoethylimidazoles with pharmaceutical activity against fungi and bacteria are known from D.O.S. 1,940,388 and from J. Med. Chem., *12*, 790 (1969) and *18*, 531 (1975).

The compounds of formula (I) show a very advantageous spectrum of microbicidal activity, rendering them useful for the protection of crops without causing undesired side-effects.

Examples of crops within the scope of the invention are cereals, maize, rice, vegetables, sugar-beet, soybeans, groundnuts, fruit-trees, ornamentals, grapevines, hops, cucurbitaceae (gherkins, cucumbers, melons), solanaceae such as potatoes, tobacco and tomatoes, as well as bananas, cocoa and rubber.

The compounds of formula (I) can be used to reduce or destroy fungal growth on plants of these or related crops or on parts of such plants (e.g., fruits, blossoms, foliage, stams, tubers, roots), whereby the newly outgrowing parts of such plants are also protected against fungal attack. The compounds of this invention are active against phytopathogenic fungi belonging to the following classes: Ascomycetes (e.g. Erysiphaceae, Fusarium, Helminthosporium); Basidiomycetes such as particularly rust-fungi (e.g. Puccinia,

Tilletia); Fungi imperfecti (e.g. Moniliales etc., Cercospora, Botrytis and Piricularia). Moreover, the compounds of formula (I) exert a systemic action. They can further be used as seed-dressings for the treatment of seed (e.g. fruits, tubers, grains) and cuttings to protect them from fungal infection and against fungi occuring in the soil.

The compounds of formula (I) show particularly promising because of their long-lasting activity, and the fact that, until now no phytotoxic effects of the active substances could be demonstrated.

Consequently the invention also comprises the use of compounds of formula (I) for combating plant diseases, in particular fungal diseases of plants.

Preferred as plant-fungicides are compounds of formula (I), their salts and metal complexes, wherein

a) $R_1$ and $R_2$ are both chloro,

b) $R_1$ is hydrogen and $R_2$ is phenyl,

c) $R_3$ is $C_1$—$C_8$-alkyl, $C_5$—$C_8$-cycloalkyl or benzyl, optionally substituted on the phenyl nucleus with halo or nitro, and $R_4$ is hydrogen,

d) $R_3$ and $R_4$, together with the nitrogen atom to which they are attached form a morpholine radical which is optionally substituted with $C_1$—$C_2$-alkyl.

Particularly preferred are those compounds which combine the characteristics of groups a) to d): thus compounds of formula (I) wherein:

aa) $R_1$ and $R_2$ are chloro, $R_4$ is hydrogen, and $R_3$ is $C_1$—$C_8$-alkyl, $C_5$—$C_8$-cycloalkyl or benzyl, optionally substituted on the phenyl nucleus with halo or nitro;

bb) $R_1$ and $R_2$ are chloro and $R_3$ and $R_4$ together with the nitrogen atom to which they are attached represent a morpholine ring which is optionally substituted with $C_1$—$C_2$-alkyl;

cc) $R_1$ is hydrogen, $R_2$ is phenyl and $R_3$ and $R_4$ together with the nitrogen atom to which they are attached represent a morpholine ring which is optionally substituted with $C_1$—$C_2$-alkyl.

As preferred species are to be considered:

2′-(2,4-dichlorophenyl)-2′-N-morpholinoethyl-(1)-1H-1,2,4-triazole,

2′-(2,4-dichlorophenyl)-2′-(4-chlorobenzylamino)-ethyl-(1)-1H-1,2,4-triazole, and

2′-(2,4-dichlorophenyl)-2′-cyclopentylaminoethyl-(1)-1H-1,2,4-triazole.

The 2′-amino-2′-phenylethyl-1H-1,2,4-triazoles of the formula (I) are prepared, according to the invention, by the reaction of an ω-1H-1,2,4-triazol-1-yl-acetophenone of the formula (II)

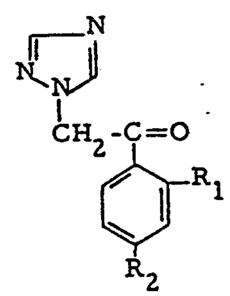

(II)

with an amine of the formula (III)

$$H—NR_3R_4 \qquad (III)$$

and subsequently hydrogenating in a manner known per se the thus obtained enamine of the formula

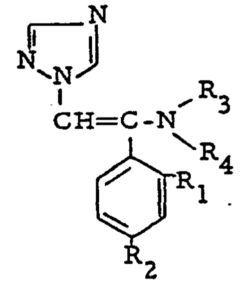

(IV)

wherein $R_1$ to $R_4$ have the above-indicated meaning, or, when $R_4$ is hydrogen, the isomeric azomethine of the formula

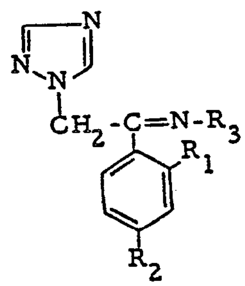

(V)

The compounds of the formula (I) wherein $R_3$ is methyl or hydrogen and $R_4$ is hydrogen, can further be prepared according to the invention by the reaction of an ω-1H-1,2,4-triazol-1-yl-acetophenone of the formula (II) with formamide

$$HCONH_2 \qquad (VI)$$

and subsequently hydrolysing the thus formed formamide of the formula (VII)

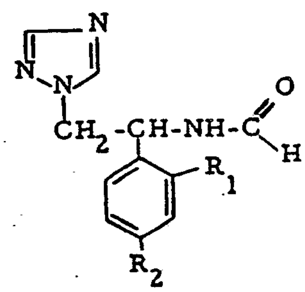

(VII)

in a manner known per se to obtain a free amine of the formula (Ia)

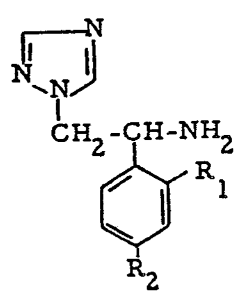

(Ia)

or reducing said (VII) in a manner known per se into a methylamine of the formula (Ib), whereby $R_1$ and $R_2$ have in the intermediate formulas (Ia) and (Ib) the same meaning as in formula (I).

The compounds of formula (I) can alternatively be prepared according to the invention by reacting a compound of the formula (Ia), optionally in a stepwise manner, with alkylating agents of the formulas VIII and IX.

$$R_3X \text{ (VIII)}, \qquad R_4Y \text{ (IX)}$$

wherein $R_3$ and $R_4$ have the same meaning as indicated for formula (I), and X and Y are, independently from each other, leaving groups.

The reaction of II with III is preferably conducted in inert organic solvents such as, for example, aromatic hydrocarbons, such as benzene, toluene, xylene, mesitylene; chlorinated hydrocarbons such as

chloroform, tetrachloromethane, dichloromethane and chlorinated aromatic hydrocarbons such as chlorobenzene.

The condensation reaction of (II) with (III) is advantageously carried out in the presence of a catalytic amount of a Lewis acid, such as, for example, p-toluenesulfonic acid, borotrifluoride, aluminum chloride and zinc chloride.

It is further advantageous to remove the water which is formed during the reaction from the reaction mixture, e.g. by azeotropic distillation or by adding a water-decomposing agent.

The reaction temperatures are from 50 to 200°C, preferably from 100 to 150°C. It is appropriate to heat the reaction mixture at reflux.

When the compounds of formula (I) are obtained as bases, they may be transformed in the corresponding salts by reacting them with inorganic or organic acids, or in metal complexes of formula (I) by their reaction with equimolar amounts of metal salts. Inversely, salts of formula (I) can be converted into the free bases of formula (I), e.g. by their reaction with alkali-hydrogencarbonate or alkali hydroxide.

The enamines or azomethines of formula (IV) and (V) may be hydrogenated with elementary hydrogen, e.g. using a nobel metal contact-catalyst such as palladium or platinum. Particular reducing agents for enamines are e.g. sodium cyanoborohydride, and for azomethines, e.g. sodium borohydride, $BH_3.HN(CH_3)_2$, sodium, sodium amalgam or kathodic reduction.

The compounds of formula (I) according to the invention possess an asymmetric centre in their carbon atom 2. Since this asymmetry emerges during a non-stereospecific reaction (hydrogenation), the compounds are usually available as racemates and they are used as such. In case further asymmetric centres occur in one or both of the radicals $R_3$ and $R_4$ there are formed diastereomeric mixtures of compounds of formula I which may be separated into their enantiomers according to known methods.

The reduction of the formamides of formula (VII) is advantageously carried out with lithium aluminumhydride in an inert organic solvent, such as, for example, ethers such as diethylether, dioxane and tetrahydrofuran.

As leaving groups X and Y there may be chosen: halo, preferably chloro, bromo and iodo; sulfonic acid radicals such as benzenesulfonic, toluenesulfonic, p-bromobenzenesulfonic, methanesulfonic, trifluoromethanesulfonic; sulfuric and phosphoric acid radicals.

The sequence of reactions carried out (ketone → enamine/azomethine → amine) is described in the literature in J. Med. Chem. *12*, 790 (1969) and *18*, 531 (1975).

The triazolylacetophenones of formula (II), used as starting materials are partly described as fungicides in D.O.S. Nos. 2,431,407 and 2,610,022 or they may be prepared analogously. Amines of the formula (III) are commercially available.

A further subject of the invention is that it provides a new process for the preparation of ω-1H-1,2,4-triazolyl-1-ylacetophenones of formula (II).

These compounds are obtained with both greater purity and better yield than in the process of D.O.S. Nos. 2,431,407 and 2,610,022, when a ketal of the formula (X)

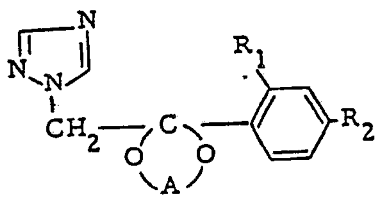

(X)

wherein $R_1$ and $R_2$ are as indicated for formula (I) and A stands for an optionally by $C_1$—$C_4$-alkyl substituted $C_2$—$C_3$ alkylene bridge, is hydrolysed in the presence of acids in a manner known per se.

Appropriate acids are mineral acids such as, for example, hydrochloric acid, sulfuric acid, nitric acid, phosphoric acid, hydrobromic acid or organic acids such as, formic, acetic, propionic, trifluoroacetic, methanesulfonic or toluenesulfonic acid.

The starting materials of formula (X) are known from U.S. Pat. No. 4,160,838.

The enamines of formula (IV) and azomethines of formula (V), prepared as intermediate products, are novel and possess furthermore fungicidal properties. Consequently they constitute a further feature of the subject invention.

The active ingredients of formula (I) may be applied to surfaces or plants to be treated together with or in subsequent order with other active substances. These active substances may be fertilizers, trace element suppliers or plant growth regulating agents. They may however also be herbicides, insecticides, bactericides, nematicides, molluscicides or mixtures of such agents, optionally together with carriers, tensides or other aiding substances for the administration which are used in the formulation art.

Appropriate carriers and additives in compositions according to the invention may be solid or liquid and correspond with substances usually employed in the art of formulating, such as, for example, natural or regenerated mineral substances, solvents, dispersants, wetting agents, adhesives thickeners, binders or fertilizers.

As tensides are considered surface active substances which are mostly dissolved or dispersed in one liquid and which are preferably adsorbed on surfaces. A tenside molecule possesses at least one group with high affinity for highly polar substances — which in general gives rise to the water-solubility — and at least another group with low affinity for water. Tensides are thus molecules with a lipophilic = hydrophobic, i.e. water-repellant or fat-attractant part, usually a bridging group with alkyl and/or aryl components, and additionally with a hydrophilic = lipophobic, i.e. water-attractant and fat-repellent part, e.g. a perfluoroalkyl radical. The products usually employed in practice are mostly mixtures of such products. Tensides not only enable a fine distribution of the active substance in a liquid, e.g. aqueous medium, but also improve the wettability of the plants. This results in a reduction of the amount of active substance in the agent to be used and consequently in a lighter load on the environment.

The invention comprises such agents for use in agriculture which contain at least one compound of formula (I) as an active substance.

The preparation of agents according to the invention is performed in a manner known per se by intimately mixing and grinding the active substances of formula (I) together with appropriate carriers and/or fillers, optionally while adding inert anti-foaming agents, wetting agents, dispersants and/or solubilizers. The active substances may occur in the following formulation forms and may be applied accordingly:

Solid forms: dusts, granulates, coated granulates, impregnated and homogenous granulates;

Water-dispersible concentrates: spray powders (wettable powders), pastes, emulsions, emulsifiable concentrates and suspension concentrates (flowables)

Liquid forms: solutions.

The content of active substance in the above agents is from 0.1 to 95%, preferably from 1 to 80%. The forms may be diluted from there to 0.001%. The employed doses are in general from 0.01 to 10 kg of active substance pro ha, preferably 0.025 to 5 kg/ha.

In the following examples temperatures are indicated in centigrades. Parts and percentages relate to weight, pressures are indicated in millibar (mb)(1 mb = 100 Pascal).

Examples of chemical preparation:

Example I

a) *Preparation of intermediate.*

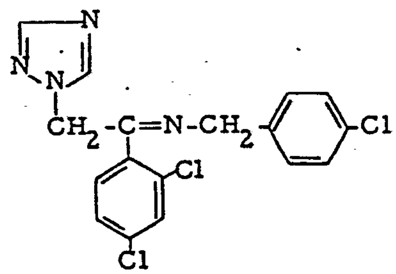

(compound 210)

2'-(2,4-dichlorophenyl)-2'-(4-chlorobenzyl-imino)-ethyl-(1)-1H-1,2,4-triazole.

To a boiling solution of 13 g ω-1Hl-1,2,4-triazol-1-yl-2,4-dichloroacetophenone in 150 ml xylene are added dropwise and slowly 10.5 g of 4-chlorobenzylamine. The formed water is distilled off with the solvent. After 4 hours the reaction is completed and the reaction mixture is evaporated in vacuo. There are obtained 18.3 g of crude 2'-(2,4-dichlorophenyl)-2'-(4-chlorobenzyl-imino)-ethyl-(1)-1H-1,2,4-triazole as a viscous brown-yellow oil.

b) *Preparation of final product.*

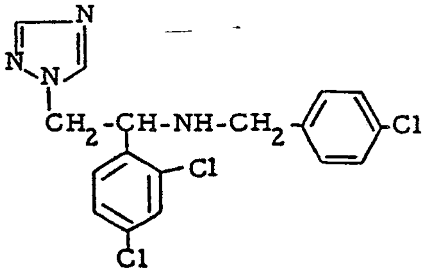

(compound 24)

2'-(2,4-dichlorophenyl)-2-(4-chlorobenzyl-amino)-ethyl-(1)-1H-1,2,4-triazole.

To a cooled (10°C) solution of 18.3 g of crude 2'-(2,4-dichlorophenyl)-2'-(4-chlorobenzylimino)-ethyl-(1)-1H-1,2,4-triazole in 150 ml of methanol is added dropwise a solution of 2.5 g sodium borohydride in 30 ml methanol. Upon completion, the reaction mixture is warmed for 2 hours at 50°C. After dilution of the mixture with ice-water, the methanol is extracted in vacuo and purified by column-chromatography on silica gel, using ethyl acetate as a solvent. After evaporation of the eluent there are obtained 9.4 g of 2′-(2,4-dichlorophenyl)-2′-(4-chlorobenzyl-amino)-ethyl-(1)-1H-1,2,4-triazole as a yellowish oil, boiling point 195—197°C/0.07 mb.

Example II

a) *Preparation of intermediate.*

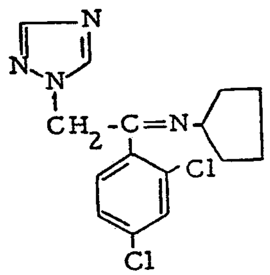

(compound 206)

2′-(2,4-dichlorophenyl)-2′-cyclopentylimino-ethyl-(1)-1H-1,2,4-triazole.

To a boiling solution of 13 g ω-1H-1,2,4-triazol-1-yl-2,4-dichloroacetophenone in 150 ml xylene are added dropwise and slowly 6.3 g cyclopentylamine. The formed water is distilled off with the solvent. There are obtained 17.2 g of crude 2′-(2,4-dichlorophenyl)-2′-cyclopentylimino-ethyl-(1)-1H-1,2,4-triazole as a viscous oil.

b) *Preparation of final product.*

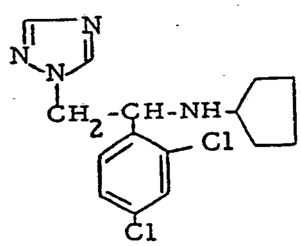

(compound 15)

2′-(2,4-dichlorophenyl)-2′-cyclopentylamino-ethyl-(1)-1H-1,2,4-triazole.

To a cooled (10°C) solution of 17.2 g of crude 2′-(2,4-dichlorophenyl)-2′-cyclopentylimino-ethyl-(1)-1H-1,2,4-triazole in 150 ml methanol is added dropwise a solution of 2.5 g sodium borohydride in 30 ml methanol. Upon completion the reaction mixture is warmed at 50°C for 2 hours. After dilution of the reaction mixture with ice-water, the methanol is evaporated in vacuo, the residue is extracted with ethyl acetate and purified by column-chromatography on silica gel using ethyl acetate. After evaporation of the eluent there are obtained 9.0 g of 2′-(2,4-dichlorophenyl)-2′-cyclopentylamino-ethyl-(1)-1H-1,2,4-triazole as a yellowish oil.

Example III

a) *Preparation of intermediate.*

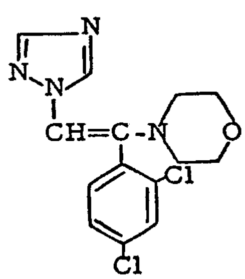

(compound 107)

2-(2,4-dichlorophenyl)-2-N-morpholino-1-(1H-1,2,4-triazol-1-yl)ethene.

To a gently boiling solution of 35 g ω-1H-1,2,4-triazol-1-yl-2,4-dichloroacetophenone in 200 ml xylene

are added dropwise and slowly 27 ml morpholine. The formed water is distilled off with the solvent. After 6 hours the reaction is completed. The cooled reaction mixture is taken up with water and ether. After 3 washings with water the organic phase is evaporated. The residue is crystallized from hexane/diethylether (2:1), yielding 28 g of 2-(2,4-dichlorophenyl)-2-N-morpholino-1-(1H-1,2,4-triazol-1-yl)-ethene as brown crystals, mp. 126—127°C.

b) *Preparation of final product.*

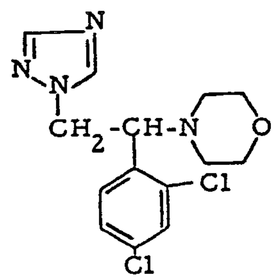

(compound 40)

2'-(2,4-dichlorophenyl)-2'-N-morpholino-ethyl-(1)-1H-1,2,4-triazole.

To a cooled (5°C) solution of 14.6 g 2-(2,4-dichlorophenyl)-2-N-morpholino-1-(1H-1,2,4-triazol-1-yl)-ethene and 6.9 ml trifluoroacetic acid in 50 ml tetrahydrofuran is added dropwise while stirring a solution of 1.9 g sodium cyanoborohydride in 30 ml methanol. After further stirring for 5 hours at 25°C the solution is evaporated, alkalized with a 2N sodium hydroxide solution and twice extracted with ether. Evaporation and vacuum distillation of the remaining oily residue yields 10 g 2'-(2,4-dichlorophenyl)-2'-N-morpholino-ethyl-(1)-1H-1,2,4-triazole, b.p. 205—208°C/0.05 mb.

Example IV

a) *Preparation of intermediate.*

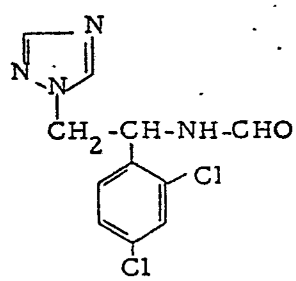

2'-(2,4-dichlorophenyl)-2'-formylamino-ethyl-(1)-1H-1,2,4-triazole.

To a mixture of 120 ml formamide, 5 ml formic acid and 51 g ω-1H-1,2,4-triazol-1-yl-2,4-dichloroaceto-phenone which has been heated and stirred for 2 hours at 155—160°C, is added dropwise during 8 hours a mixture of 20 ml formamide and 10 ml formic acid while the same temperature is kept. After cooling the reaction mixture is poured into ice-water. The precipitate 2'-(2,4-dichlorophenyl)-2'-formylamino-ethyl-(1)-1H-1,2,4-triazole is crystallized from ethyl acetate. Yield 32 g; m.p. 206—208°C.

b) *Preparation of intermediate or final product.*

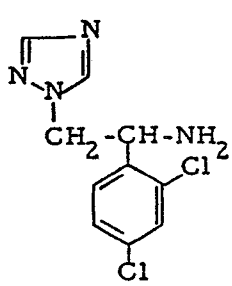

(compound 1)

2'-(2,4-dichlorophenyl)-2'-amino-ethyl-(1)-1H-1,2,4-triazole.

25 g 2'-(2,4-dichlorophenyl)-2'-formylimino-ethyl-(1)-1H-1,2,4-triazole are boiled under reflux for 3

hours in 150 ml of 10% hydrochloric acid. The clear solution is diluted with ice-water and alkalized with concentrated sodium hydroxide solution. The precipitated 2'-(2,4-dichlorophenyl)-2'-amino-ethyl-(1)-1H-1,2,4-triazole is recrystallized from ethyl acetate. Yield 21 g; m.p. 108—110°C.

c) *Preparation of final product.*

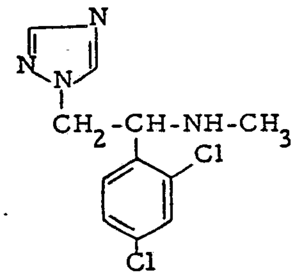

(compound 2)

2'-(2,4-dichlorophenyl)-2'-methylamino-ethyl-(1)-1H-1,2,4-triazole

To a solution of 5.7 g 2'-(2,4-dichlorophenyl)-2'-formylamino-ethyl-(1)-1H-1,2,4-triazole in 60 ml absolute tetrahydrofuran are added portionwise under cooling 1.2 g lithiumaluminum hydride. After heating at reflux for 3 hours, 1 ml of tert. butanol is added and after 30 further minutes 150 ml of water. The resulting mixture is concentrated in vacuo, the residue is extracted with diethylether, the organic phase is evaporated and distilled in vacuo. There are obtained 2.9 g 2'-(2,4-dichlorophenyl)-2'-methylamino-ethyl-(1)-1H-1,2,4-triazole as a colorless distillate, with bp. 185—190°C/0.01 mb, which crystallizes upon cooling. M.p. 51—52°C.

## Example V

*Preparation of starting material.*

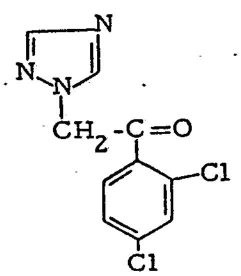

ω-1H-1,2,4-triazol-1-yl-2,4-dichloroacetophenone.

350 g 1-[2-(2,4-dichlorophenyl)-1,3-dioxolan-2-ylmethyl]-1H-1,2,4-triazole are boiled under reflux for 48 hours in 1800 ml 20% hydrochloric acid. After cooling, the solution is diluted with 2000 ml water, alkalized with concentrated sodium hydroxide and extracted with methylene chloride. Evaporation of the organic phase and recrystallization from chloroform yields 275 g ω-1H-1,2,4-triazol-1-yl-2,4-dichloroacetophenone, mp. 112—115°C.

The compounds prepared according to Examples 1 to 4 or prepared in an analogous manner are listed in the following tables.

TABLE 1
Compounds of Formula (I)

| No. | $R_1$ | $R_2$ | $R_3$ | $R_4$ | phys. data |
|---|---|---|---|---|---|
| 1 | Cl | Cl | H— | H | mp. 108—110°C |
| 2 | Cl | Cl | $CH_3$— | H | mp. 51—52°C |
| 3 | Cl | Cl | n-$C_3H_7$— | H | visc. oil |
| 4 | Cl | Cl | n-$C_4H_9$— | H | visc. oil |
| 5 | Cl | Cl | i-$C_4H_9$— | H | visc. oil |
| 6 | Cl | Cl | s-$C_4H_9$— | H | visc. oil |
| 7 | Cl | Cl | neo-$C_5H_{11}$— | H | visc. oil |
| 8 | Cl | Cl | i-$C_5H_{11}$— | H | visc. oil |
| 9 | Cl | Cl | n-$C_6H_{13}$— | H | visc. oil |
| 10 | Cl | Cl | n-$C_8H_{17}$— | H | visc. oil |
| 11 | H | H | $CH_3$—$CH_2$—$C(CH_3)_2$— | H | bp. 180—185°C/0.05 mb |
| 12 | H | H | cyclo-$C_3H_5$— | H | visc. oil |
| 13 | Cl | Cl | cyclo-$C_3H_5$— | H | visc. oil |
| 14 | H | Br | cyclo-$C_3H_5$— | H | visc. oil |
| 15 | Cl | Cl | cyclo-$C_5H_9$— | H | bp. 165—170°C/0.05 mb |
| 16 | Cl | Cl | cyclo-$C_6H_{11}$— | H | visc. oil |
| 17 | H | $C_6H_5$ | cyclo-$C_6H_{11}$— | H | mp. 95—97°C |
| 18 | Cl | Cl | 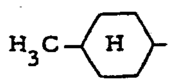 | H | visc. oil |
| 19 | Cl | Cl | 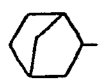 | H | visc. oil |

TABLE 1
Compounds of Formula (I) (cont'd.)

| No. | $R_1$ | $R_2$ | $R_3$ | $R_4$ | phys. data |
|---|---|---|---|---|---|
| 20 | Cl | Cl | cyclo-$C_8H_{15}$— | H | visc. oil |
| 21 | Cl | Cl | $(CH_3)_2N—CH_2—CH(CH_3)$— | H | visc. oil |
| 22 | Cl | Cl | n-$C_4H_9—O—(CH_2)_3$— | H | — |
| 23 | Cl | Cl | $C_6H_5—CH_2$— | H | bp. 185—187°C/0.05 mb |
| 24 | Cl | Cl | 4-Cl—$C_6H_4—CH_2$— | H | bp. 195—197°C/0.07 mb |
| 25 | Cl | Cl | 2-Cl-4-Cl—$C_6H_3—CH_2$— | H | mp. 80—81°C |
| 26 | Cl | Cl | $C_6H_5—CH_2—CH_2$— | H | — |
| 27 | Cl | Cl | $C_6H_5—CH(CH_3)$— [S] | H | visc. oil |
| 28 | Cl | Cl | $C_6H_5—CH(CH_3)$— [R] | H | visc. oil |
| 29 | Cl | Cl | n-$C_4H_9$— | n-$C_4H_9$— | visc. oil |
| 30 | Cl | Cl | $CH_3$— | cyclo-$C_6H_{11}$— | — |
| 31 | H | H | —$(CH_2)_4$— | | visc. oil |
| 32 | H | H | —$(CH_2)_5$— | | mp. 96—97°C |
| 33 | H | Cl | —$(CH_2)_5$— | | mp. 91—94°C |
| 34 | H | Cl | —$(CH_2)_6$— | | mp. 77—80°C |
| 35 | H | H | —$(CH_2)_2—CH(CH_3)—(CH_2)_2$— | | mp. 68—70°C |
| 36 | H | Cl | —$(CH_2)_2—N(CH_3)—(CH_2)_2$— | | mp. 99—101°C |
| 37 | H | H | —$(CH_2)_2—N(CH_3)—(CH_2)_2$— | | — |
| 38 | H | H | —$(CH_2)_2—O—(CH_2)_2$— | | — |
| 39 | H | Cl | —$(CH_2)_2—O—(CH_2)_2$— | | — |
| 40 | Cl | Cl | —$(CH_2)_2—O—(CH_2)_2$— | | bp. 205—208°C/0.05 mb |
| 41 | Cl | Cl | —$CH_2—CH(CH_3)—O—CH(CH_3)—CH_2$— | | highly visc. oil |
| 42 | H | $C_6H_5$ | —$CH_2—CH(CH_3)—O—CH(CH_3)—CH_2$— | | mp. 130—140°C (HCl-salt) |

TABLE 2
Intermediates of Formula (IV)

| No. | $R_1$ | $R_2$ | $R_3$ | $R_4$ | phys. data |
|---|---|---|---|---|---|
| 101 | H | H | $—(CH_2)_4—$ | | mp. 100—102°C |
| 102 | H | H | $—(CH_2)_2—O—(CH_2)_2$ | | mp. 96—97°C |
| 103 | H | H | $—(CH_2)_2—N(CH_3)—(CH_2)_2—$ | | mp. 143—145°C |
| 104 | H | H | $—(CH_2)_2—N(CH_3)—(CH_2)_2—$ | | mp. 162—163°C |
| 105 | H | H | $—(CH_2)_5—$ | | mp. 70—71°C |
| 106 | H | Cl | $—(CH_2)_2—O—(CH_2)_2—$ | | mp. 108—110°C |
| 107 | Cl | Cl | $—(CH_2)_2—O—(CH_2)_2—$ | | mp. 126—127°C |
| 108 | H | Cl | $—(CH_2)_5—$ | | mp. 95—98°C |

TABLE 3
Intermediates of Formula (V)

| No. | $R_1$ | $R_2$ | $R_3$ | phys. data |
|---|---|---|---|---|
| 201 | Cl | Cl | n-$C_4H_9$— | visc. oil |
| 202 | Cl | Cl | neo-$C_5H_{11}$— | visc. oil |
| 203 | Cl | Cl | i-$C_5H_{11}$— | visc. oil |
| 204 | Cl | Cl | cyclo-$C_3H_5$— | visc. oil |
| 205 | Cl | Cl | cyclo-$C_6H_{11}$— | resin |
| 206 | Cl | Cl | cyclo-$C_5H_9$— | visc. oil |
| 207 | H | H | cyclo-$C_3H_5$— | oil |
| 208 | Cl | Cl | $(CH_3)_2N—CH_2—CH(CH_3)—$ | oil |
| 209 | Cl | Cl | $C_6H_5—CH_2—$ | oil |
| 210 | Cl | Cl | 4-Cl—$C_6H_4—CH_2—$ | visc. oil |
| 211 | Cl | Cl | 2-Cl-4-Cl—$C_6H_3—CH_2—$ | visc. oil |
| 212 | Cl | Cl | cyclo-$C_8H_{15}$— | oil |
| 213 | Cl | Cl | $C_6H_5—CH_2—CH_2—$ | — |
| 214 | Cl | Cl | $C_6H_5—CH(CH_3)—$ [S] | visc. oil |
| 215 | Cl | Cl | $C_6H_5—CH(CH_3)—$ [R] | visc. oil |
| 216 | Cl | Cl | 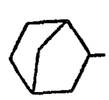 | visc. oil |

Formulation Examples.

Example VI

*Dusts*:

The following substances are used to prepare a) 5% and b) a 2% dust:

a) 5 parts of active substance
95 parts of talc;

b) 2 parts of active substance
1 part of highly dispersed silicic acid
97 parts of talc.

The active substances are mixed with the carriers and ground and in this form can be processed to dusts for application.

Example VII

*Granulate*:

The following substances are used to prepare a 5% granulate:

5 parts of active substance
0.25 parts of epoxidised vegetable oil
0.25 parts of cetyl polyglycol ether
3.25 parts of polyethylene glycol
91 parts of kaolin (particle size 0.3—0.8 mm.).

The active substance is mixed with epoxidised vegetable oil and the mixture is dissolved in 6 parts of 2-propanone. Then polyethylene glycol and cetyl polyglycol ether are added. The resultant solution is sprayed on kaolin and the 2-propanone is evaporated in vacuo. Such a micro-granulate is advantageously used for combating soil fungi.

Example VIII

*Wettable powders*:

The following constituents are used to prepare a) a 70%, b) a 40%, c) and d) a 25% and e) a 10% wettable powder:

a) 70 parts of active substance
5 parts of sodium dibutylnaphthylsulfonate
3 parts of naphthalenesulfonic acid/phenolsulfonic acid/formaldehyde condensate (3:2:1).
10 parts of kaolin
12 parts of Champagne chalk.

b) 40 parts of active substance
5 parts of sodium ligninsulfonate
1 part of sodium dibutylnaphthalenesulfonic acid
54 parts of silicic acid.

c) 25 parts of active substance
4.5 parts of calcium ligninsulfonate
1.9 parts of Champagne chalk/hydroxyethyl cellulose mixture (1:1).
1.5 parts of sodium dibutylnaphthalenesulfonate
19.5 parts of silicic acid
19.5 parts of Champagne chalk
28.1 parts of kaolin.

d) 25 parts of active substance
2.5 parts of isooctylphenoxy-polyethylene-ethanol
1.7 parts of a Champagne chalk/hydroxyethyl cellulose mixture (1:1)
8.3 parts of sodium aluminum silicate
16.5 parts of kieselguhr
46 parts of kaolin

e) 10 parts of active substance
3 parts of a mixture of the sodium salts of saturated fatty alcohol sulfates
5 parts of naphthalenesulfonic acid/formaldehyde condensate
82 parts of kaolin.

The active substances are intimately mixed in suitable mixers with the additives and ground in appropriate mills and rollers.

Wettable powders of excellent wettability and suspension power are obtained. These wettable powders can be diluted with water to give suspensions of the desired concentration and can be used in particular for leaf application.

Example IX

*Emulsifiable concentrates*:

The folowing substances are used to prepare a 25% emulsifiable concentrate:

25 parts of active substance

2.5 parts of epoxidised vegetable oil
10 parts of an alkylarylsulfonate/fatty alcohol polyglycol ether mixture
5 parts of dimethyl formamide
57.5 parts of dimethylbenzene.

By diluting such a concentrate with water it is possible to prepare emulsions of the desired concentration, which are especially suitable for leaf application.

Example X

Activity against Puccinia graminis on wheat.

a) *Residual protective action*:

Wheat plants where sprayed 6 days after sowing with a spray broth (0.06% of active substance) prepared from a wettable powder of the active substance. After 24 hours the treated plants were infected with a suspension of Uredospores of the fungus. After an incubation period of 48 hours at 95—100% relative humidity and at about 20°C the plants were stood in a greenhouse at approx. 22°C. The development of rust-pestules was evaluated 12 days after the infection.

The compounds of formula (I) were fully active. At the low concentration of 0.002%, compounds 15, 24 and 40 inter alia were still fully active.

b) *Systemic action*:

5 Days after sowing wheat plants are sprayed with a spray broth (containing 0.006% of active substance; the amount of the spray being proportional with the soil-volume) prepared from a wettable powder of the active substance. After 48 hours the treated plants are infected with a suspension of Uredospores of the fungus. After an incubation period of 48 hours at 95—100% relative humidity and at 20°C the treated plants are stood in a glass-house at about 22°C. The rust-pustules are evaluated 12 days after the day of infection.

Part of the compounds of formula (I), e.g. compounds 2, 3, 5, 15 and 40 completely prevented fungal disease through their systemic action.

Certain compounds of formula (I), e.g. compounds 3, 5 and 15 completely prevented fungal attack even at concentrations as low as 0.0006%.

Example XI

Activity against Cercospora personata (= C. arachidicola) on peanut plants.

3-week-old peanut plants were sprayed with a spray (0.02% of the active substance) prepared from a wettable powder of the active principle. After about 12 h the treated plants were dusted with a conidium suspension of the fungus. The infected plants were incubated for about 24 h at 90% relative humidity and then placed in a greenhouse at about 22°C. The incidence of the fungus infection was evaluated after 12 days.

In comparison with the untreated controls, plants that had been treated with the active principles of formula (I) displayed little or no fungal infestation.

Compounds 2, 3, 24 and 40, inter alia, completely prevented the disease at a concentration of 0.02%.

Compounds 3 and 24 completely prevented fungal attack at a concentration of 0.002% also.

Example XII

Activity against Erysiphe graminis on barley.

a) *Residual protective action*:

Barley plants, about 8 cm in height, are sprayed with a spray broth (containing 0.02% of active substance) prepared from a wettable powder of the active substance. After 3—4 hours the treated plants are dusted with conidia of the fungus. The infected barley plants are then placed in a glass-house at about 22°C and fungal attack is evaluated 10 days after the day of infection.

b) *Systemic action*:

A spray broth (containing 0.006% of the active substance; the amount being proportional with the soil-volume), prepared from a wettable powder of the active substance is administered to barley plants, about 8 cm in height, while care is taken that the external parts of the plants do not enter into contact with the spray. After 48 hours the treated plants are dusted with conidia of the fungus. The infected barley-plants are stood in a glass-house at 22°C and the fungal infection is evaluated after 10 days.

*Experiment a)*:

The compounds of formula (I) showed complete activity. (no disease). Compounds 2, 5, 8, 15 and 24 inter alia still showed complete activity at a concentration of 0.002%.

*Experiment b)*:

The compounds 2, 3, 5, 8, 15, 24 and 40 inter alia showed complete activity. The compounds of formula (I) consequently show an excellent activity in the prevention of mildew. The compounds 2, 3, 5, 8, 15 and 40 were still completely active at a concentration of 0.0006%.

Example XIII

Residual-protective action against Venturia inaequalis on apple shoots.

Apple saplings with fresh shoots of 10—20 cm were sprayed with a spray (0.06% of the active substance) prepared from a wettable powder of the active ingredient. After 24 hours the treated plants were infected with a conidium suspension of the fungus. The plants were then incubated for 5 days at 90—100% relative humidity and kept for 10 further days in a glass-house at 20—24°C. The incidence of scab is determined 15 days after the infection.

The compounds of formula (I) showed complete activity at the indicated concentration of 0.06%. The compounds 24 and 40 inter alia were still fully active (no disease) at the lower concentration of 0.02%.

**Claims**

1. A compound selected from the group consisting of a 2'-amino-2'-phenyl-ethyl-1H-1,2,4-triazole of the formula (I)

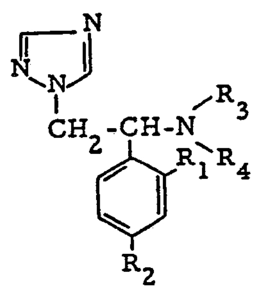

(I)

and the phytopharmaceutically acceptable salts and metal complexes thereof, wherein:

$R_1$ is a member selected from the group consisting of hydrogen, halo, $C_1$—$C_4$-alkyl, $C_1$—$C_4$-alkyloxy, and $C_1$—$C_2$-haloalkyl;

$R_2$ is a member selected from the group consisting of hydrogen, halo, $C_1$—$C_4$-alkyl, $C_1$—$C_4$-alkyloxy, $C_1$—$C_2$-haloalkyl and phenyl;

$R_3$ is a member selected from the group consisting of hydrogen, $C_1$—$C_{12}$-alkyl, $C_3$—$C_8$-cycloalkyl which is optionally substituted with $C_1$—$C_4$-alkyl, and aralkyl with maximum 2 carbon atoms in the alkyl part and optionally substituted on the phenyl nucleus with halo or nitro;

$R_4$ is a member selected from the group consisting of hydrogen and $C_1$—$C_4$-alkyl; whereby

$R_3$ and $R_4$ may, together with the nitrogen atom to which they are attached, form a 3- to 7-membered saturated heterocyclic ring which may optionally be interrupted by oxygen, sulfur or $NR_5$ and optionally substituted with $R_6$, wherein $R_5$ and $R_6$ are each independently $C_1$—$C_4$-alkyl.

2. A compound according to claim 1 wherein said $R_1$ and $R_2$ are both chloro.

3. A compound according to claim 1 wherein said $R_1$ is hydrogen and said $R_2$ is phenyl.

4. A compound according to claim 1 wherein said $R_3$ is selected from the group consisting of $C_1$—$C_8$-alkyl, $C_5$—$C_8$-cycloalkyl, and benzyl which is optionally substituted on the phenyl nucleus with halo or nitro; and $R_4$ is hydrogen.

5. A compound according to claim 1 wherein said $R_3$ and $R_4$ together with the nitrogen atom to which they are attached form a morpholine radical which is optionally substituted with $C_1$—$C_2$-alkyl.

6. A compound according to claim 2 wherein said $R_4$ is hydrogen and said $R_3$ is selected from the group consisting of $C_1$—$C_8$-alkyl, $C_5$—$C_8$-cycloalkyl and benzyl which is optionally substituted on the phenyl nucleus with halo or nitro.

7. A compound according to claim 2 wherein said $R_3$ and $R_4$ together with the nitrogen atom to which they are attached form a morpholine radical which is optionally substituted with $C_1$—$C_2$-alkyl.

8. A compound according to claim 3 wherein said $R_3$ and $R_4$ together with the nitrogen atom to which they are attached form a morpholine radical which is optionally substituted with $C_1$—$C_2$-alkyl.

9. A compound selected from the group consisting of 2'-(2,4-dichlorophenyl)-2'-N-morpholino-ethyl-(1)-1H-1,2,4-triazole and the phytopharmaceutically acceptable salts and metal complexes thereof.

10. A compound selected from the group consisting of 2'-(2,4-dichlorophenyl)-2'-(4-chlorobenzyl-amino)-ethyl-(1)-1H-1,2,4-triazole and the phytopharmaceutically acceptable salts and metal complexes thereof.

11. A compound selected from the group consisting of 2'-(2,4-dichlorophenyl)-2'-cyclopentylamino-ethyl-(1)-1H-1,2,4-triazole and the phytopharmaceutically acceptable salts and metal complexes thereof.

12. A compound of the formula

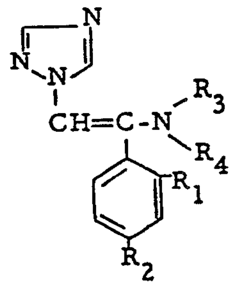

(IV)

wherein $R_1$, $R_2$, $R_3$ and $R_4$ are as defined in claim 1.

13. A compound of the formula

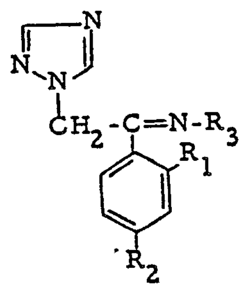

(V)

wherein $R_1$, $R_2$ and $R_3$ are as defined in claim 1.

14. A process of preparing a 2'-amino-2'-phenyl-ethyl-1H-1,2,4-triazole of the formula (I), according to claim 1 which comprises reacting an ω-1H-1,2,4-triazol-1-yl-acetophenone of the formula (II)

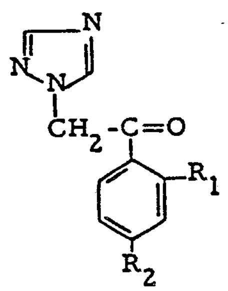

(II)

with an amine of the formula (III)

$$H—NR_3R_4 \quad (III)$$

and subsequently hydrogenating in a known per se maner the thus formed enamine of the formula (IV)

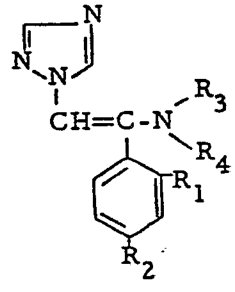

(IV)

wherein $R_1$, $R_2$, $R_3$ and $R_4$ are as defined in formula (I), or in case $R_4$ is hydrogen, the isomeric azomethine of the formula (V)

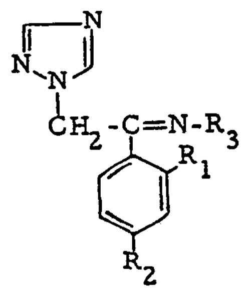

(V)

15. A process of preparing a compound of the formula (Ia)

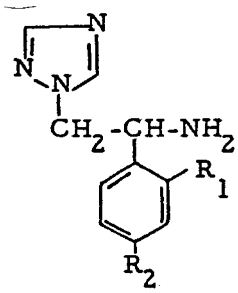

(Ia)

or a compound of the formula (Ib)

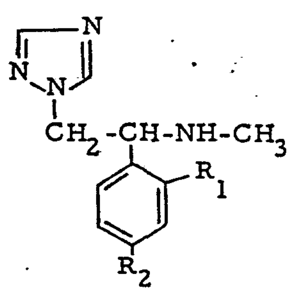

(Ib)

wherein $R_1$ and $R_2$ are as defined in claim 1, which comprises reacting an 1H-1,2,4-triazol-1-yl-acetophenone of the formula (II) with formamide

$$H—CO—NH_2 \qquad (VI)$$

in the presence of formic acid to form a formamide of the formula (VII)

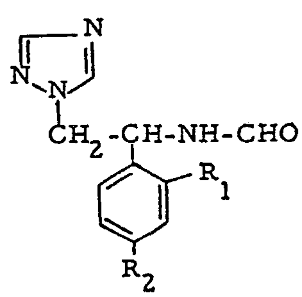

(VII)

wherein $R_1$ and $R_2$ are as defined in formula (I), and subsequently either hydrolyzing said (VII) in a known manner to produce a free amine of the formula (Ia), or, reducing said (VII) in a known manner to produce a methylamine of the formula (Ib).

16. A process of preparing a compound of formula (I), according to claim 1, which comprises reacting in a known manner, optionally stepwise, a compound of the formula (Ia) as defined in claim 15 with alkylating agents of the formulas (VIII) and (IX),

$R_3X$ , $R_4Y$
(VIII) (IX)

wherein $R_3$ and $R_4$ are as defined in formula (I) and X and Y are, independently from each other, leaving groups.

17. A microbicidal agent comprising inert carriers and/or additives and as an active ingredient a compound of formula (I) as defined in claim 1.

18. A microbicidal agent according to claim 18, comprising a compound according to any of the claims 2 to 11.

19. A microbicidal agent according to claim 18, comprising a compound according to one of the claims 12 and 13.

20. A process of preparing an agent according to one of the claims 17 to 19 which comprises intimately mixing or grinding the active ingredient with the carriers and/or other additives.

21. A method of combating microorganisms which comprises the use of a compound of formula (I).

22. A method of combating microorganisms according to claim 21 which comprises the use of a compound as claimed in any of claims 2 to 11.

23. A method of combating microorganisms which comprises the use of a compound as claimed in any of the claims 12 and 13.

24. A method of combating microorganisms according to any of claims 21 to 23 wherein said microorganisms are fungi, in particular plant-pathogenic fungi.

**Patentansprüche**

1. Eine Verbindung, ausgewählt aus der Gruppe, die aus einem 2'-Amino-2'-phenyl-ethyl-1H-1,2,4-triazol mit der Formel (I)

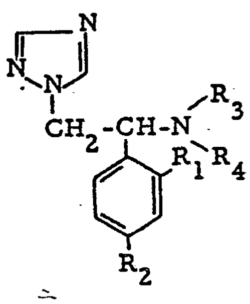

(I)

und den phytopharmazeutisch annehmbaren Salzen und Metallkomplexen hievon besteht, worin:

$R_1$ ein aus der aus Wasserstoff, Halogen, $C_1$—$C_4$-Alkyl, $C_1$—$C_4$-Alkyloxy und $C_1$—$C_2$-Halogenalkyl bestehenden Gruppe ausgewähltes Glied ist;

$R_2$ ein aus der aus Wasserstoff, Halogen, $C_1$—$C_4$-Alkyl, $C_1$—$C_4$-Alkyloxy, $C_1$—$C_2$-Halogenalkyl und Phenyl bestehenden Gruppe ausgewähltes Glied ist;

$R_3$ ein aus der aus Wasserstoff, $C_1$—$C_{12}$-Alkyl, $C_3$—$C_8$-Cycloalkyl, das gegebenenfalls mit $C_1$—$C_4$-Alkyl substituiert ist, und Aralkyl mit höchstens 2 Kohlenstoffatomen im Alkylteil, das gegebenenfalls im Phenylkern durch Halogen oder Nitro substituiert ist, bestehenden Gruppe ausgewähltes Glied ist;

$R_4$ ein aus der aus Wasserstoff und $C_1$—$C_4$-Alkyl bestehenden Gruppe ausgewähltes Glied ist; wobei

$R_3$ und $R_4$ gemeinsam mit dem Stickstoffatom, an das sie gebunden sind, einen 3- bis 7-gliedrigen gesättigten heterocyclischen Ring bilden können, der gegebenenfalls durch Sauerstoff, Schwefel oder $NR_5$ unterbrochen und gegebenenfalls durch $R_6$ substituiert sein kann, worin $R_5$ und $R_6$ jeweils unabhängig $C_1$—$C_4$-Alkyl bedeuten.

2. Eine Verbindung nach Anspruch 1, worin die genannten Reste $R_1$ und $R_2$ beide Chlor bedeuten.

3. Eine Verbindung nach Anspruch 1, worin der genannte Rest $R_1$ Wasserstoff ist und der genannte Rest $R_2$ Phenyl bedeutet.

4. Eine Verbindung nach Anspruch 1, worin der genannte Rest $R_3$ aus der aus $C_1$—$C_8$-Alkyl, $C_5$—$C_8$-Cycloalkyl und Benzyl, das gegebenenfalls am Phenylkern durch Halogen oder Nitro substituiert ist, bestehenden Gruppe ausgewählt ist; und $R_4$ Wasserstoff bedeutet.

5. Eine Verbindung nach Anspruch 1, worin die genannten Reste $R_3$ und $R_4$ gemeinsam mit dem Stickstoffatom, an welches sie gebunden sind, einen Morpholinrest bilden, der gegebenenfalls durch $C_1$—$C_2$-Alkyl substituiert ist.

6. Eine Verbindung nach Anspruch 2, worin der genannte Rest $R_4$ Wasserstoff ist und der genannte Rest $R_3$ aus der aus $C_1$—$C_8$-Alkyl, $C_5$—$C_8$-Cycloalkyl und Benzyl, das gegebenenfalls am Phenylkern durch Halogen oder Nitro substituiert ist, gebildeten Gruppe ausgewählt ist.

7. Eine Verbindung nach Anspruch 2, worin die genannten Reste $R_3$ und $R_4$ gemeinsam mit dem Stickstoffatom, an welches sie gebunden sind, einen Morpholinrest ausbilden, der gegebenenfalls durch $C_1$—$C_2$-Alkyl substituiert ist.

8. Verbindung nach Anspruch 3, worin die genannten Reste $R_3$ und $R_4$ gemeinsam mit dem Stickstoffatom, an welches sie gebunden sind, einen Morpholinrest ausbilden, der gegebenenfalls durch $C_1$—$C_2$-Alkyl substituiert ist.

9. Eine Verbindung, ausgewählt aus der aus 2'-(2,4-Dichlorphenyl)-2'-N-morpholino-ethyl(1)-1H-1,2,4-triazol und den phytopharmazeutisch annehmbaren Salzen und Metallkomplexen hievon bestehenden Gruppe.

10. Eine Verbindung, ausgewählt aus der aus 2'-(2,4-Dichlorphenyl)-2'-(4-chlorbenzylamino)-ethyl-(1)-1H-1,2,4-triazol und den phytopharmazeutisch annehmbaren Salzen und Metallkomplexen hievon bestehenden Gruppe.

11. Eine Verbindung, ausgewählt aus der aus 2'-(2,4-Dichlorphenyl)-2'-cyclopentylamino-ethyl-(1)-1H-1,2,4-triazol und den phytopharmazeutisch annehmbaren Salzen und Metallkomplexen hievon bestehenden Gruppe.

12. Eine Verbindung der Formel

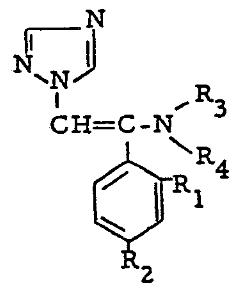

(IV)

worin $R_1$, $R_2$, $R_3$ und $R_4$ wie in Anspruch 1 definiert sind.

13. Eine Verbindung der Formel

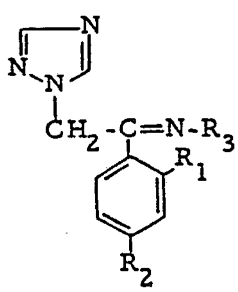

(V)

worin $R_1$, $R_2$ und $R_3$ wie in Anspruch 1 definiert sind.

14. Ein Verfahren zur Herstellung eines 2'-Amino-2'-phenylethyl-1H-1,2,4-triazols der Formel (I) gemäß Anspruch 1, welches ein Umsetzen eines ω-1H-1,2,4-Triazol-1-yl-acetophenons der Formel (II)

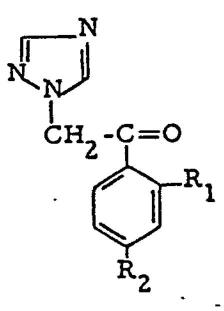

(II)

mit einem Amin der Formel (III)

$$H—NR_3R_4 \quad (III)$$

und ein anschließendes, in an sich bekannter Weise erfolgendes Hydrieren des solcherart gebildeten Enamins der Formel (IV)

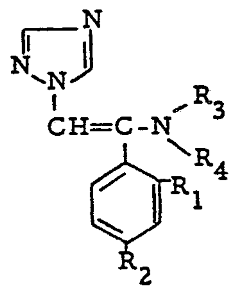

(IV)

worin $R_1$, $R_2$, $R_3$ und $R_4$ wie in Formel (I) definiert sind, oder im Falle, daß $R_4$ Wasserstoff bedeutet, des isomeren Azomethins der Formel (V)

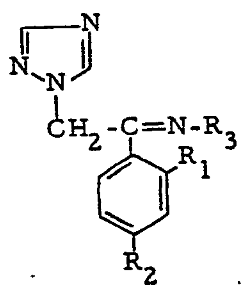

(V)

umfaßt.

15. Ein Verfahren zur Herstellung einer Verbindung der Formel (Ia)

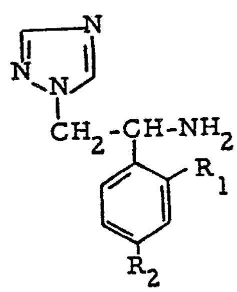

(Ia)

oder einer Verbindung der Formel (Ib)

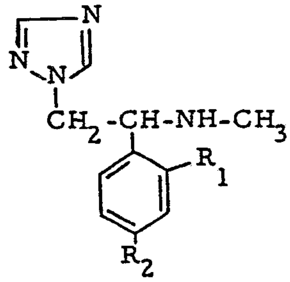

(Ib)

worin $R_1$ und $R_2$ wie in Anspruch 1 definiert sind, welches ein Umsetzen eines 1H-1,2,4-Triazol-1-yl-acetophenons der Formel (II) mit Formamid

$$H—CO—NH_2 \qquad (VI)$$

in Gegenwart von Ameisensäure unter Ausbildung eines Formamids der Formel (VII)

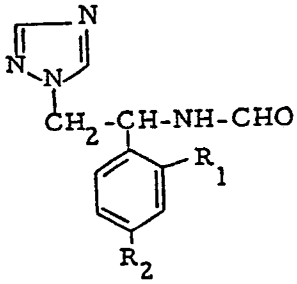

(VII)

worin $R_1$ und $R_2$ wie in Formel (I) definiert sind, und anschließend entweder ein Hydrolysieren dieser Verbindung (VII) in an sich bekannter Weise zur Ausbildung eines freien Amins der Formel (Ia) oder ein Reduzieren dieser Verbindung (VII) in bekannter Weise unter Ausbildung eines Methylamins der Formel (Ib) umfaßt.

16. Verfahren zur Herstellung einer Verbindung der Formel (I) nach Anspruch 1, welches ein in bekannter Weise erfolgendes, gewünschtenfalls stufenweises Umsetzen einer Verbindung der Formel (Ia), wie in Anspruch 15 definiert, mit Alkylierungsmitteln der Formeln (VIII) und (IX)

$$\underset{(VIII)}{R_3X}\,, \qquad \underset{(IX)}{R_4Y}\,,$$

worin $R_3$ und $R_4$ wie in Formel (I) definiert sind und X und Y unabhängig voneinander Leaving-Gruppen bedeuten, umfaßt.

17. Ein mikrobicides Mittel, umfassend inerte Träger und/oder Additive und als einen wirksamen Bestandteil eine Verbindung der Formel (I), wie in Anspruch 1 definiert.

18. Ein mikrobicides Mittel nach Anspruch 17, umfassend eine Verbindung nach einem der Ansprüche 2 bis 11.

19. Mikrobicides Mittel nach Anspruch 17, umfassend eine Verbindung nach einem der Ansprüche 12 und 13.

20. Ein Verfahren zur Herstellung eines Mittels gemäß einem der Ansprüche 17 bis 19, welches ein inniges Vermischen oder Vermahlen des wirksamen Bestandteiles mit den Trägern und/oder anderen Additiven umfaßt.

21. Ein Verfahren zur Bekämpfung von Mikroorganismen, welches die Anwendung einer Verbindung der Formel (I) umfaßt.

22. Ein Verfahren zur Bekämpfung von Mikroorganismen nach Anspruch 21, welches die Anwendung einer Verbindung, wie in einem der Ansprüche 2 bis 11 beansprucht, umfaßt.

23. Ein Verfahren zur Bekämpfung von Mikroorganismen, welches die Anwendung einer Verbindung, wie in einem der Ansprüche 12 und 13 beansprucht, umfaßt.

24. Ein Verfahren zur Bekämpfung von Mikroorganismen nach einem der Ansprüche 21 bis 23, wobei die genannten Mikroorganismen Pilze, insbesondere pflanzenpathogene Pilze sind.

**Revendications**

1. Un composé choisi dans le groupe constitué par un 2′-amino-2′-phényléthyl-1H-1,2,4-triazole de formule (I)

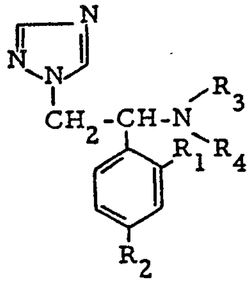

(I)

et leurs sels et complexes métalliques convenant en phytopharmacie où:

$R_1$ est un composant du groupe constitué par un hydrogène, un halogéno, un alkyle en $C_1$—$C_4$, un alcoxy en $C_1$—$C_4$ et un halogénoalkyle en $C_1$—$C_2$;

$R_2$ est un composant du groupe constitué par un hydrogène, un halogéno, un alkyle en $C_1$—$C_4$, un alcoxy en $C_1$—$C_4$, un halogénoalkyle en $C_1$—$C_2$ et un phényle;

$R_3$ est un composant du groupe constitué par l'hydrogène, un alkyle en $C_1$—$C_{12}$, un cycloalkyle en $C_3$—$C_8$, qui est éventuellement substitué par un alkyle en $C_1$—$C_4$, et un aralkyle avec au maximum 2 atomes de carbone dans la portion alkyle et éventuellement substitué sur le noyau phényle par un halogéno ou un nitro;

$R_4$ est un composant du groupe constitué par l'hydrogène et un alkyle en $C_1$—$C_4$; et

$R_3$ et $R_4$ peuvent ensemble, avec l'atome d'azote auquel ils sont fixés, former un hétérocycle saturé triangulaire à heptagonal qui peut éventuellement être interrompu par l'oxygène, le soufre ou $NR_5$ et éventuellement substitué par $R_6$, où $R_5$ et $R_6$ sont chacun indépendamment un alkyle en $C_1$—$C_4$.

2. Un composé selon la revendication 1, dans lequel lesdits $R_1$ et $R_2$ sont tous deux un chloro.

3. Un composé selon la revendication 1, dans lequel ledit $R_1$ est un hydrogène et ledit $R_2$ est un phényle.

4. Un composé selon la revendication 1, dans lequel ledit $R_3$ est choisi dans le groupe constitué par un alkyle en $C_1$—$C_8$, un cycloalkyle en $C_5$—$C_8$ et un benzyle qui est éventuellement substitué sur le noyau phényle par un halogéno ou un nitro; et $R_4$ est un hydrogène.

5. Un composé selon la revendication 1, où lesdits $R_3$ et $R_4$, ensemble avec l'atome d'azote auquel ils sont fixés, forment un radical morpholine qui est éventuellement substitué par un alkyle en $C_1$—$C_2$.

6. Un composé selon la revendication 2, dans lequel ledit $R_4$ est un hydrogène et ledit $R_3$ est choisi dans le groupe constitué par un alkyle en $C_1$—$C_8$, un cycloalkyle en $C_5$—$C_8$ et un benzyle qui est éventuellement substitué sur le noyau phényle par un halogéno ou un nitro.

7. Un composé selon la revendication 2, dans lequel lesdits $R_3$ et $R_4$, ensemble avec l'atome d'azote auquel ils sont fixés, forment un radical morpholine qui est éventuellement substitué par un alkyle en $C_1$—$C_2$.

8. Un composé selon la revendication 3, dans lequel lesdits $R_3$ et $R_4$, ensemble avec l'atome d'azote auquel ils sont fixés, forment un radical morpholine qui est éventuellement substitué par un alkyle en $C_1$—$C_2$.

9. Un composé choisi dans le groupe constitué par le 2'-(2,4-dichlorophényl)-2'-N-morpholinoéthyl-(1)-1H-1,2,4-triazole et ses sels et complexes métalliques convenant en phytopharmacie.

10. Un composé choisi dans le groupe constitué par le 2'-(2,4-dichlorophényl)-2'-(4-chlorobenzylamino)-éthyl-(1)-1H-1,2,4-triazole et ses sels et complexes métalliques convenant en phytopharmacie.

11. Un composé choisi dans le groupe constitué par le 2'-(2,4-dichlorophényl)-2'-cyclopentylamino-éthyl-(1)-1H-1,2,4-triazole et ses sels et complexes métalliques convenant en phytopharmacie.

12. Un composé de formule

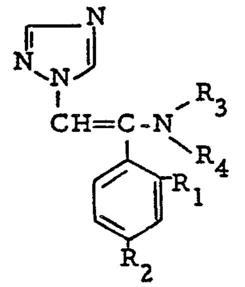

(IV)

dans laquelle $R_1$, $R_2$, $R_3$ et $R_4$ sont comme défini dans la revendication 1.

13. Un composé de formule

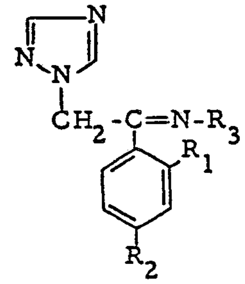

(V)

où $R_1$, $R_2$ et $R_3$ sont comme défini dans la revendication 1.

14. Un procédé de préparation d'un 2'-amino-2'-phényléthyl-1H-1,2,4-triazole de formule (I) selon la revendication 1 qui comprend la réaction d'une ω-1H-1,2,4-triazole-1-yl-acétophénone de formule (II)

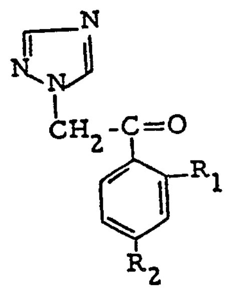

(II)

avec une amine de formule (III)

$$H—NR_3R_4 \qquad (III)$$

puis l'hydrogénation de façon connue en soi de l'énamine ainsi formée de formule (IV)

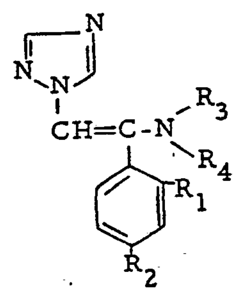

(IV)

où $R_1$, $R_2$, $R_3$ et $R_4$ sont comme défini dans la formule (I) ou, dans le cas où $R_4$ est un hydrogène, de l'azométhine isomère de formule (V)

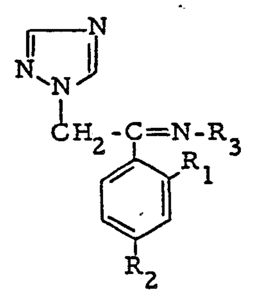

(V)

15. Un procédé de préparation d'un composé de formule (Ia)

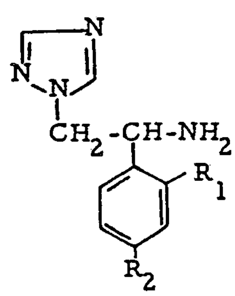

(Ia)

ou d'un composé de formule (Ib)

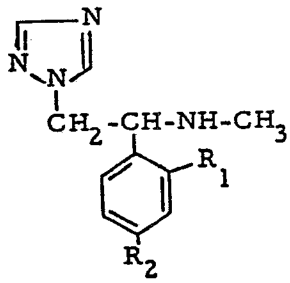

(Ib)

où $R_1$ et $R_2$ sont comme défini dans la revendication 1, qui comprend la réaction d'une 1H-1,2,4-triazole-1-yl-acétophénone de formule (II) avec le formamide

$$H—CO—NH_2 \quad (VI)$$

en présence d'acide formique pour former un formamide de formule (VII)

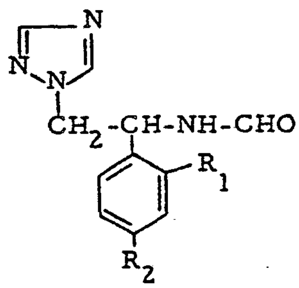

(VII)

où $R_1$ et $R_2$ sont comme défini dans la formule (I) puis soit l'hydrolyse dudit (VII) de façon connue pour produire une amine libre de formule (Ia), soit la réduction dudit (VII) de façon connue pour produire une méthylamine de formule (Ib).

16. Un procédé de préparation d'un composé de formule (I) selon la revendication 1, qui comprend la réaction de façon connue, éventuellement graduelle, d'un composé de formule (Ia) comme défini dans la revendication 15, avec des agents d'alkylation de formules (VIII) et (IX),

$$\underset{(VIII)}{R_3X}\ , \qquad \underset{(IX)}{R_4Y}$$

où $R_3$ et $R_4$ sont comme défini dans la formule (I) et X et Y sont indépendamment l'un de l'autre des groupes labiles.

17. Un agent microbicide comprenant des supports et/ou des additifs inertes et comme ingrédient actif un composé de formule (I) comme défini dans la revendication 1.

18. Un agent microbicide selon la revendication 17, comprenant un composé selon l'une quelconque des revendications 2 à 11.

19. Un agent microbicide selon la revendication 17, comprenant un composé selon l'une des revendications 12 et 13.

20. Un procédé pour la préparation d'un agent selon l'une des revendications 17 à 19, qui comprend le mélange ou le broyage intime de l'ingrédient actif avec les supports et/ou les autres additifs.

21. Un procédé de lutte contre les micro-organismes, qui comprend l'emploi d'un composé de formule (I).

22. Un procédé de lutte contre les micro-organismes selon la revendication 21, qui comprend l'emploi d'un composé comme revendiqué dans l'une quelconque des revendications 2 à 11.

23. Un procédé de lutte contre les microorganismes, qui comprend l'emploi d'un composé comme revendiqué dans l'une des revendications 12 et 13.

24. Un procédé de lutte contre les microorganismes, selon l'une quelconque des revendications 21 à 23 dans lequel lesdits micro-organismes sont des champignons, en particulier des champignons pathogènes pour les plantes.